Europäisches Patentamt

European Patent Office

Office européen des brevets

(11) Veröffentlichungsnummer: **0 195 451 B1**

(19)

(12) **EUROPÄISCHE PATENTSCHRIFT**

(45) Veröffentlichungstag der Patentschrift: **03.06.92**

(21) Anmeldenummer: **86103824.8**

(22) Anmeldetag: **20.03.86**

(51) Int. Cl.⁵: **A61B 5/04**

(54) **Elektrode zur Messung von Körperströmen.**

(30) Priorität: **20.03.85 DE 3509976**

(43) Veröffentlichungstag der Anmeldung:
**24.09.86 Patentblatt 86/39**

(45) Bekanntmachung des Hinweises auf die
Patenterteilung:
**03.06.92 Patentblatt 92/23**

(84) Benannte Vertragsstaaten:
**BE DE FR GB IT NL SE**

(56) Entgegenhaltungen:
**US-A- 3 642 008**
**US-A- 3 842 394**
**US-A- 3 993 049**
**US-A- 4 112 941**

(73) Patentinhaber: **ARBO Medizin-Technologie
GmbH
Wendenstrasse 2-3
W-3300 Braunschweig(DE)**

(72) Erfinder: **Ding, Wolfgang
Berliner Strasse 5
W-3302 Cremlingen(DE)**
Erfinder: **Arnold, Werner
Georgstrasse 13
W-4053 Jüchen 1(DE)**

(74) Vertreter: **Lins, Edgar, Dipl.-Phys. et al
Patentanwälte Gramm + Lins Theodor-
Heuss-Strasse 2
W-3300 Braunschweig(DE)**

# Beschreibung

Die Erfindung betrifft eine Elektrode zur Messung von Körperströmen mit einer zur Auflage auf der Haut bestimmten elektrisch leitenden und klebenden Kontaktschicht und einer die Kontaktschicht abdeckenden Abdeckschicht sowie mit einem mit einem Übertragungskabel verbundenen metallischen Sensor, insbesondere aus einem Silbermaterial, zur Aufnahme und Weiterleitung der Ströme, der mit der Kontaktschicht in elektrisch leitender Verbindung steht.

Es ist bekannt, Elektroden mit einer elektrisch leitenden und klebenden Kontaktschicht auszustatten. Eine derartige Kontaktschicht kann aus einem viskoelastischen Gel gebildet sein, das Karaya enthält und beispielsweise in der DE-PS 27 27 396 beschrieben ist. Der Vorteil dieser Kontaktschichten besteht darin, daß sie aufgrund ihrer viskoelastischen Eigenschaften einen intimen Kontakt mit der Haut eingehen und daher einen geringen Übergangswiderstand zwischen Elektrode und Haut produzieren. Zugleich wirken sie als Befestigungsmittel, da sie klebende Eigenschaften haben.

Zur Weiterleitung der Körperströme auf ein Auswertungsgerät muß ein Sensor vorgesehen sein, der üblicherweise durch eine Silber/Silberchloridschicht gebildet ist. In einer durch eine offenkundige Vorbenutzung der Anmelderin bekannten Ausführungsform einer derartigen Elektrode liegt eine AG/AGCL-Folie mit einer Dicke von 0,02 mm auf der Kontaktschicht auf und ist mit einem durch die Abdeckschicht ragenden Druckknopf verbunden, an den das Übertragungskabel angebracht wird. Derartige Elektroden werden als Einmal-Elektroden verwendet. Ihre Kosten werden wesentlich durch das für den Sensor benötigte Silbermaterial bestimmt.

Der Erfindung liegt daher die Aufgabe zugrunde, eine Elektrode der eingangs erwähnten Art zu erstellen, die wie eine Einmal-Elektrode verwendbar, im Gebrauch preiswert sowie einfach zu handhaben ist.

Diese Aufgabe wird erfindungsgemäß bei einer Elektrode der eingangs erwähnten Art dadurch gelöst, daß die Abdeckschicht am Rand der Elektrode eine Aussparung aufweist und daß eine ansetzbare sowie abnehmbare Klammer vorgesehen ist, die mit einer ersten Backe die beim Gebrauch auf der Haut aufliegende Seite der Kontaktschicht und mit einer zweiten Backe die andere Seite der Kontaktschicht durch die Aussparung ergreift, die den Sensor auf einer bzw. den Anlageflächen der Backen aufweist und die mit dem Übertragungskabel verbunden ist.

Erfindungsgemäß besteht der nur einmal verwendbare Teil der Elektrode lediglich aus der Kontaktschicht sowie der Abdeckschicht, wobei die Abdeckschicht am Rande der Kontaktschicht eine Aussparung aufweist. Die Kontaktschicht wird mit Hilfe der Klammer durch die Aussparung der Abdeckschicht hindurch ergriffen, wobei der metallische Sensor an der Klammer vorgesehen ist.

Die Aussparung der Abdeckschicht kann beispielsweise dadurch hergestellt sein, daß die Kontaktschicht eine Lasche aufweist, die nicht von der Abdeckschicht bedeckt ist. An dieser Lasche, die leicht gegenüber dem übrigen Teil der Kontaktschicht angehoben werden kann, wird die Klammer angesetzt.

Um gut reproduzierbare Resultate und eine reproduzierbare Kontaktgabe zu erhalten, ist es vorteilhaft, wenn die die Oberseite der Kontaktschicht ergreifende zweite Backe der Klammer einen konischen Stift aufweist, wenn die Kontaktschicht mit einem Loch zur Aufnahme des konischen Stifts versehen ist und wenn die andere Backe der Klammer eine ebene Anlagefläche für die Kontaktschicht aufweist. In der ebenen Anlagefläche kann vorzugsweise der Sensor angeordnet sein. Zur Erhöhung des Andrucks ist es vorteilhaft, wenn der Sensor dabei gegenüber seiner Umgebungsfläche erhaben angeordnet ist, wodurch der durch den Sensor erzeugte Druck auf die Kontaktschicht verstärkt wird.

Der konische Stift fährt in das Loch der Kontaktschicht so weit ein, wie die Größe des Loches dies aufgrund der Konizität des Stiftes erlaubt. Dadurch ist sichergestellt, daß die zweite Backe mit dem Stift einen eindeutigen ringförmigen Kontakt zur Kontaktschicht herstellt.

In einer besonders bevorzugten Ausführungsform ist die Abdeckschicht formstabil und so vorgeformt, daß sie die Kontaktschicht an dem Bereich der Aussparung der Abdeckschicht anhebt. Dabei wird die Angriffsfläche für die Klammer in einem Abstand von der Hautoberfläche gehalten, so daß die Klammer ohne weitere Manipulationen an der auf die Haut aufgeklebten Elektrode ansetzbar ist.

Die Erfindung soll im folgenden anhand von in der Zeichnung dargestellten Ausführungsbeispielen näher erläutert werden. Es zeigen:

Figur 1    den Randbereich einer mit einer Abdeckschicht versehenen Kontaktschicht mit einer angesetzten Klammer mit einem Sensor

Figur 2    eine Darstellung wie in Figur 1 mit einer Klammer mit Zwei Sensoren

Figur 3    eine Ansicht auf eine streifenförmige Kontaktschicht mit einer die Breite der Kontaktschicht wesentlich übersteigenden Abdeckschicht von unten

Figur 4    eine Ansicht von unten auf eine Kontaktschicht in der Größe der Abdeckschicht.

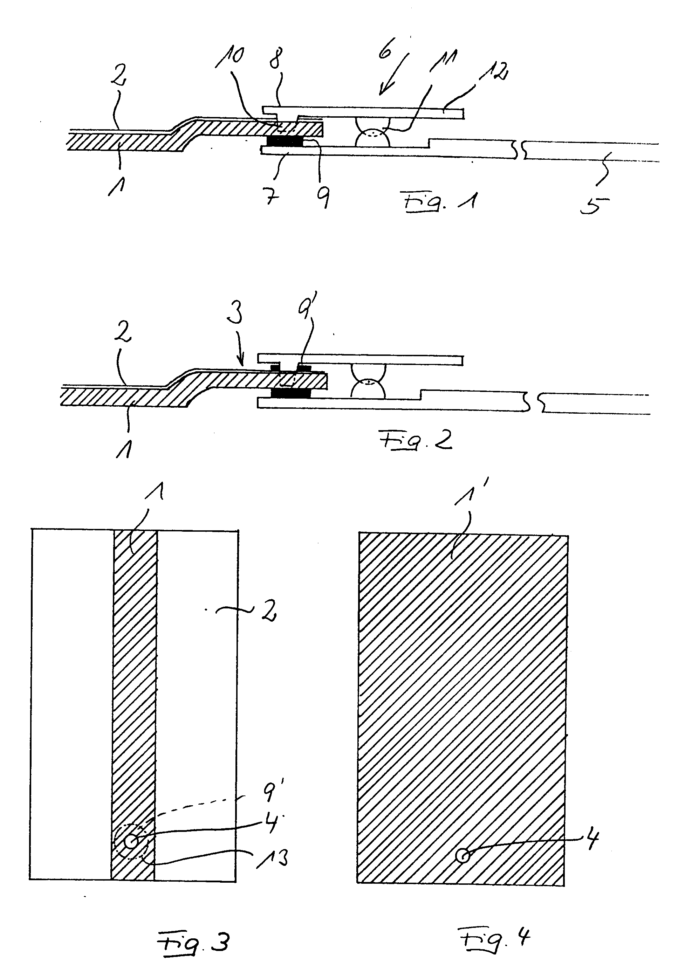

In den Figuren 1 und 2 ist der einmal verwendbare Teil einer Elektrode durch eine leitende und klebende Kontaktschicht 1 gebildet, die durch eine Abdeckfolie 2 ganzflächig bedeckt ist. Die Abdeckfolie 2 ist dabei formstabil und , beispielsweise durch Tiefziehen, vorgeformt, so daß am Ende der Kontaktschicht diese zusammen mit der Abdeckfolie 2 angehoben ist und sich parallel zur Auflagefläche erstreckt. In dem angehobenen Ende 3 weist die Abdeckschicht eine Ausnehmung in Form eines kreisförmigen Loches auf, das sich als Loch 4 der Kontaktschicht 1 fortsetzt. Die Kontaktierung zu einem Anschlußkabel 5 erfolgt über eine Klammer 6, die zwei federnd gegeneinanderdrückende Backen 7,8 aufweist. Die Backen 7,8 der Klammer 6 ergreifen die Kontaktschicht 1 im Bereich des Loches 4, wobei die Unterseite der Kontaktschicht von der unteren ersten Backe 7 ergriffen wird, die eine durch einen scheibenartigen Sensor 9 gebildete ebene Anlagefläche aufweist. Der Sensor 9 steht dabei erhaben über die Umgebungsfläche auf der Innenseite der ersten Backe 7. Die Kontaktschicht 1 wird von oben mit der zweiten Backe 8 ergriffen, die in das Loch 4 mit einem konischen Stift 10 hineingedrückt wird. Der größte Durchmesser des konischen Stifts 10 ist wesentlich größer als der Durchmesser des Loches 4, so daß der Stift 10 durch den oberen Rand des Loches 4 gehalten wird. Die beiden Backen sind an einem Drehgelenk 11 miteinander verbunden, wobei ein nach hinten über das Drehgelenk 11 hinausstehender Hebel 12 der zweiten Backe 8 als Betätigungshebel für die Klammer 6 dient.

Figur 2 verdeutlicht, daß zur Sicherung der Kontaktierung auch die obere zweite Backe 8 mit einem den konischen Stift 10 ringförmig umschließenden Sensor 9' versehen sein kann, der durch eine entsprechend erweiterte Ausnehmung der Abdeckfolie 2 gegen die Oberseite der Kontaktschicht 1 drückt.

Figur 3 zeigt eine Ausbildung der Kontaktschicht 1 und Abdeckfolie 2, in der die Kontaktschicht streifenförmig ausgebildet ist und die Abdeckfolie eine wesentlich größere Breite aufweist, so daß sie auf beiden Seiten über die Kontaktschicht 1 übersteht. Die Unterseite der Abdeckfolie ist dabei vorzugsweise mit einem Klebstoff versehen, so daß die Abdeckfolie die Klebwirkung der Kontaktschicht 1 zum Andruck der Kontaktschicht 1 an die Haut unterstützen kann.

In dem in Figur 4 dargestellten Ausführungsbeispiel nimmt dagegen die Kontaktschicht 1' die volle Fläche der (hier nicht dargestellten) Abdeckfolie 2 ein, so daß die Klebwirkung ausschließlich durch die Kontaktschicht 1' hervorgerufen wird.

In Figur 3 ist noch die Lage des Sensors 9' angedeutet, für den eine entsprechend vergrößerte Ausnehmung 13 in der Abdeckfolie 2 vorgesehen ist.

## Patentansprüche

1. Elektrode zur Messung von Körperströmen mit einer zur Auflage auf der Haut bestimmten elektrisch leitenden und klebenden Kontaktschicht (1) und einer die Kontaktschicht (1) abdeckenden Abdeckschicht (2) sowie mit einem mit einem Übertragungskabel (5) verbundenen metallischen Sensor (9, 9'), insbesondere aus einem Silbermaterial, zur Aufnahme und Weiterleitung der Ströme, der mit der Kontaktschicht (1) in elektrisch leitender Verbindung steht, **dadurch gekennzeichnet, daß** die Abdeckschicht (2) am Rand der Elektrode eine Aussparung (4, 13) aufweist und daß eine ansetzbare sowie abnehmbare Klammer (6) vorgesehen ist, die mit einer ersten Backe (7) die beim Gebrauch auf der Haut aufliegende Seite der Kontaktschicht (1) und mit einer zweiten Backe (8) die andere Seite der Kontaktschicht (1) durch die Aussparung (4, 13) ergreift, die den Sensor (9, 9') auf einer bzw. den Anlageflächen der Backen aufweist und die mit dem Übertragungskabel (5) verbunden ist.

2. Elektrode nach Anspruch 1, dadurch gekennzeichnet, daß die die Oberseite der Kontaktschicht (1) ergreifende Backe (8) der Klammer (6) einen konischen Stift (10) aufweist, daß die Kontaktschicht (1) mit einem Loch (4) zur Aufnahme des konischen Stifts (10) versehen ist und daß die andere Backe (7) der Klammer (6) eine ebene Auflagefläche für die Kontaktschicht (1) aufweist.

3. Elektrode nach Anspruch 2, dadurch gekennzeichnet, daß die Auflagefläche eben und durch den Sensor (9) gebildet ist.

4. Elektrode nach Anspruch 3, dadurch gekennzeichnet, daß der Sensor (9) gegenüber seiner Umgebungsfläche erhaben angeordnet ist.

5. Elektrode nach einem der Ansprüche 1 bis 4, dadurch gekennzeichnet, daß die Abdeckschicht (2) die Kontaktschicht (1) in dem Bereich der Aussparung (4,13) der Abdeckschicht (2) anhebt.

6. Elektrode nach Anspruch 5, dadurch gekennzeichnet, daß die Abdeckschicht formstabil und vorgeformt ist.

## Claims

1. Electrode for measuring bodily currents, having an electrically conductive and adhesive contact layer (1) for bearing on the skin, and a cover layer (2) covering the contact layer (1), and having a metal sensor (9, 9') which is connected to a transmission cable (5) and is in particular of a silver material, for receiving and passing on the currents, and is in electrically conductive connection with the contact layer (1), characterized in that the cover layer (2) has a cutout (4, 13) at the edge of the electrode, and in that a clip (6) which may be placed on and taken off is provided and grips by means of a first jaw (7) the side of the contact layer (1) lying on the skin during use and grips by means of a second jaw (8) the other side of the contact layer (1) through the cutout (4, 13), has the sensor (9, 9') on one or the contact surfaces of the jaws and is connected to the transmission cable (5).

2. Electrode according to Claim 1, characterized in that the jaw (8) of the clip (6) gripping the upper side of the contact layer (1) has a conical pin (10), in that the contact layer (1) is provided with a hole (4) for receiving the conical pin (10), and in that the other jaw (7) of the clip (6) has a planar bearing surface for the contact layer (1).

3. Electrode according to Claim 2, characterized in that the bearing surface is planar and is formed by the sensor (9).

4. Electrode according to Claim 3, characterized in that the sensor (9) is arranged to be raised with respect to the surface surrounding it.

5. Electrode according to one of Claims 1 to 4, characterized in that the cover layer (2) raises the contact layer (1) in the region of the cutout (4, 13) of the cover layer (2).

6. Electrode according to Claim 5, characterized in that the cover layer is dimensionally stable and pre-shaped.

**Revendications**

1. Electrode de mesure de signaux physiologiques comportant une couche de contact (1) conductrice électrique et adhésive destinée à être posée sur la peau et une couche de recouvrement (2) qui recouvre la couche de contact (1), ainsi qu'un capteur métallique (9, 9'), en particulier en un matériau argentique, connecté à un câble de transmission (5) et destiné à capter et transmettre les signaux, qui est en liaison conductrice électrique avec la couche de contact (1), caractérisée en ce que la couche de recouvrement (2) présente, au bord de l'électrode, une lacune (4, 13) et qu'est prévue une pince (6) pouvant être mise en place et retirée qui, par une première mâchoire (7), attaque la face de la couche de contact (1) posée sur la peau lors de l'utilisation et, par une seconde mâchoire (8), l'autre face de la couche de contact (1), à travers la lacune (4, 13) que présente le capteur (9, 9') sur une face d'appui ou les faces d'appui des mâchoires et qui est connectée au câble de transmission (5).

2. Electrode suivant la revendication 1, caractérisée en ce que la mâchoire (8) de la pince (6) qui attaque la face supérieure de la couche de contact (1) présente un téton conique (10), que la couche de contact (1) est pourvue d'un trou (4) destiné à recevoir le téton conique (10) et que l'autre mâchoire (7) de la pince (6) présente une surface d'appui plane pour la couche de contact (1).

3. Electrode suivant la revendication 2, caractérisée en ce que la surface d'appui est plane et est formée par le capteur (9).

4. Electrode suivant la revendication 3, caractérisée en ce que le capteur (9) est surélevé par rapport à la surface qui l'environne.

5. Electrode suivant l'une quelconque des revendications 1 à 4, caractérisée en ce que la couche de recouvrement (2) soulève la couche de contact (1) dans la région de la lacune (4, 13) de la couche de recouvrement (2).

6. Electrode suivant la revendication 5, caractérisée en ce que la couche de recouvrement est de forme stable et est préfaçonnée.

Fig. 1

Fig. 2

Fig. 3

Fig. 4